Europäisches Patentamt

(19) European Patent Office

Office européen des brevets

(11) EP 0 749 747 B1

(12) **FASCICULE DE BREVET EUROPEEN**

(45) Date de publication et mention de la délivrance du brevet:
**16.12.1998 Bulletin 1998/51**

(51) Int. Cl.⁶: **A61K 7/035**, A61K 7/48, A61K 7/00

(21) Numéro de dépôt: **96401312.2**

(22) Date de dépôt: **17.06.1996**

(54) **Composition comprenant une dispersion de particules de polymères dans un milieu non aqueux**

Zusammensetzung die eine Dispersion von Polymerpartikeln in nichtwässrigem Medium enthält

Composition containing a dispersion of polymer particles in a non aqueous medium

(84) Etats contractants désignés:
**AT BE CH DE ES FR GB IT LI NL SE**

(30) Priorité: **21.06.1995 FR 9507428**

(43) Date de publication de la demande:
**27.12.1996 Bulletin 1996/52**

(73) Titulaire: **L'OREAL**
**75008 Paris (FR)**

(72) Inventeurs:
• **Mougin, Nathalie**
**75011 Paris (FR)**
• **Mondet, Jean**
**93600 Aulnay sous Bois (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
EP-A- 0 409 690          EP-A- 0 447 286
EP-A- 0 486 394          EP-A- 0 502 769
WO-A-95/09874            FR-A- 2 689 010
GB-A- 1 202 796

• CHEMICAL ABSTRACTS, vol. 89, no. 22, 27 Novembre 1978 Columbus, Ohio, US; abstract no. 185917h, page 363; XP002013313 & JP-A-53 094 041 (KOBAYASHI KOSE CO,LTD) 17 Août 1978
• Encyclopedia of Chemical Technology Kirk Othmer, 3rd ed., 1983, vol. 21, p. 377-401

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention a trait à une composition cosmétique, pharmaceutique ou hygiénique, comprenant une dispersion de particules de polymère dispersées dans un milieu non aqueux, ainsi qu'à l'utilisation d'une telle dispersion dans une composition cosmétique, pharmaceutique ou hygiénique.

Il est connu d'utiliser en cosmétique certaines dispersions de particules de polymère de taille nanométrique, dans des milieux organiques tels que des alcools inférieurs ou des hydrocarbures aromatiques ou aliphatiques. Les polymères sont alors utilisés le plus souvent comme agent filmogène dans des produits de maquillage tels que des mascaras, des eye-liners, des ombres à paupières ou des vernis à ongles. Les compositions cosmétiques obtenues après incorporation des ces dispersions de particules de polymère ne présentent pas toujours une stabilité satisfaisante.

On connaît également dans la demande de brevet JP-A-78 94041 des compositions de maquillage à base d'une dispersion organique de polymère dans un milieu constitué de monoalcools inférieurs ou d'hydrocarbures aliphatiques ou aromatiques.

On connaît également dans la demande WO-A-95/09074 des dispersions de polymères acryliques dans un milieu alcoolique stabilisées par un copolymère-bloc à base de polyméthacrylate de méthyle et de polyacrylate de tertiobutyle.

La demanderesse a découvert de manière surprenante de nouvelles dispersions de particules de polymère stabilisées par des agents stabilisants particuliers, que l'on définira par la suite, dans de nombreux types de milieu non-aqueux.

La présente invention a pour but de proposer une dispersion de particules qui restent à l'état de particules élémentaires, sans former d'agglomérats, lorsqu'elles sont en dispersion dans les milieux non aqueux.

Un objet de la présente invention est donc une composition comprenant, dans un milieu cosmétiquement, pharmaceutiquement et/ou hygiéniquement acceptable, une dispersion de particules d'au moins un polymère stabilisé en surface par un agent stabilisant dans un milieu non aqueux, caractérisé par le fait que:

A) le milieu non aqueux est constitué d'au moins un composé liquide non aqueux choisi dans le groupe constitué par:

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges,

et

B)

- (i) lorsque le milieu non aqueux comprend au moins une huile de silicone, l'agent stabilisant est choisi dans le groupe constitué par les copolymères blocs greffés ou séquencés comprenant au moins un bloc de type polyorganosiloxane et au moins un bloc d'un polymère radicalaire ou d'un polyéther ou d'un polyester,
- (ii) lorsque le milieu non aqueux ne comprend pas une huile de silicone, l'agent stabilisant est choisi dans le groupe constitué par :

    - (a) les copolymères blocs greffés ou séquencés comprenant au moins un bloc de type polyorganosiloxane et au moins un bloc d'un polymère radicalaire ou d'un polyéther ou d'un polyester,
    - (b) les copolymères d'acrylates ou de méthacrylates d'alcools en $C_1$-$C_4$, et d'acrylates ou de méthacrylates d'alcools en $C_8$-$C_{30}$,
    - (c) les copolymères blocs greffés ou séquencés comprenant au moins un bloc résultant de la polymérisation de diènes, hydrogéné ou non-hydrogéné, et au moins un bloc d'un polymère vinylique ou acrylique ou d'un polyéther ou d'un polyester, ou leurs mélanges.

Un autre objet de l'invention est l'utilisation de ladite dispersion de particules dans et pour la préparation d'une composition cosmétique, hygiénique ou pharmaceutique.

Un avantage de la présente invention est qu'il est ainsi possible de calibrer à volonté la taille des particules de polymère, ainsi que de moduler leur polydispersité en taille lors de la synthèse. Ceci n'est pas possible lorsque l'on utilise des pigments sous forme particulaire, leur constitution ne permettant pas de moduler la taille moyenne des particules, ni leur dureté.

Un autre avantage de l'invention est que l'on peut ainsi obtenir des particules de polymère de très petite taille, notamment nanométrique, ce qui n'est pas le cas avec, par exemple, d'autres types de particules telles que des microsphères dont le diamètre est généralement supérieur à 1 micron. Cette taille importante de l'ordre du micron, a pour inconvénient d'entraîner une certaine visibilité des particules à l'oeil, lorsqu'elles sont dans une composition et/ou lorsqu'elles sont appliquées sur la peau, ainsi qu'une mauvaise stabilité de la composition comprenant ladite dispersion.

Ainsi, un autre avantage de l'utilisation de la dispersion selon l'invention est de permettre l'obtention d'une composition stable, qui peut être transparente, translucide ou opaque, selon la taille des particules de polymère qui y sont dispersées.

Les dispersions selon l'invention sont donc constituées de particules, généralement sphériques, d'au moins un polymère stabilisé en surface, dans un milieu non aqueux.

Ces dispersions peuvent notamment se présenter sous forme de nanoparticules de polymères en dispersion stable dans un milieu non aqueux. Les nanoparticules sont de préférence d'une taille comprise entre 5 et 600 nm, étant donné qu'au-delà d'environ 600 nm, les dispersions de particules deviennent beaucoup moins stables.

Les polymères utilisés dans la présente demande peuvent être de toute nature. On peut ainsi employer des polymères radicalaires, des polycondensats, voire des polymères d'origine naturelle. Le polymère peut être choisi par l'homme du métier en fonction de ses propriétés, selon l'application ultérieure souhaitée pour la composition. Ces polymères peuvent être en particulier réticulés.

Ainsi, il est possible d'utiliser des polymères filmogènes, de préférence ayant une température de transition vitreuse (Tg) basse, inférieure ou égale à la température ambiante. On obtient ainsi une dispersion qui peut filmifier lorsqu'elle est appliquée sur un support-substrat, ce qui n'est pas le cas lorsque l'on utilise des dispersions de pigments minéraux selon l'art antérieur.

Il est également possible d'utiliser des polymères non filmogènes, éventuellement réticulés, qui pourront être utilisés en tant que charges dispersées de manière stable dans le milieu non aqueux. Cette utilisation constitue également un objet de l'invention.

Les polymères utilisables dans le cadre de la présente invention ont de préférence un poids moléculaire moyen en nombre de l'ordre de 2000 à 10000000, et une température de transition vitreuse de -100°C à 300°C.

Parmi les polymères filmogènes non réticulés, on peut citer des homopolymères ou des copolymères radicalaires, acryliques ou vinyliques, de préférence ayant une Tg inférieure ou égale à 30°C.

Il est possible d'ajouter à la dispersion de polymères, un plastifiant de manière à abaisser la Tg des polymères utilisés. Le plastifiant peut être choisi parmi les plastifiants usuellement utilisés dans le domaine d'application et notamment parmi les composés susceptibles d'être des solvants du polymère.

Parmi les polymères non filmogènes, on peut citer des homopolymères ou copolymères radicalaires, vinyliques ou acryliques, éventuellement réticulés, ayant de préférence une Tg supérieure ou égale à 40°C, tels que le polyméthacrylate de méthyle, le polystyrène ou le polyacrylate de tertiobutyle.

Les composés liquides du milieu non aqueux de la dispersion de polymères doivent être liquides à température ambiante.

Le paramètre de solubilité global $\delta$ à global selon l'espace de solubilité de HANSEN est défini dans l'article "Solubility parameter values" de Eric A. Grulke de l'ouvrage "Polymer Handbook" 3$^{\text{ème}}$ édition, Chapitre VII, pages 519-559 par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc...).

La définition des solvants dans l'espace de solubilité tridimensionnel selon HANSEN est décrite dans l'article de C. M. HANSEN : "The three dimensionnal solubility parameters" J. Paint Technol. 39, 105 (1967).

Parmi les composés liquides non aqueux ayant un paramètre de solubilité global selon l'espace de solubilité de HANSEN inférieur ou égal à 17 (MPa)$^{1/2}$, on peut citer les corps gras liquides, notamment les huiles, qui peuvent être choisies parmi les huiles naturelles ou synthétiques, carbonées, hydrocarbonées, fluorées et/ou siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les hydrocarbures et notamment des huiles de paraffine, de vaseline, ou le polyisobutylène hydrogéné, l'isododécane, ou encore les 'ISOPARS', isoparaffines volatiles.

On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

On peut également utiliser un milieu non aqueux constitué d'un solvant seul ou d'un mélange de solvant, choisi parmi :

- les esters linéaires, ramifiés ou cycliques, ayant plus de 6 atomes de carbone,
- les éthers ayant plus de 6 atomes de carbone,
- les cétones ayant plus de 6 atomes de carbone.

Par monoalcools ayant un paramètre de solubilité global selon l'espace de solubilité de HANSEN inférieur ou égal à 20 $(MPa)^{1/2}$, on entend les monoalcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Le choix du milieu non aqueux est effectué par l'homme du métier en fonction de la nature des monomères constituant le polymère et/ou de la nature du stabilisant, comme indiqué ci-après.

D'une manière générale, la dispersion selon l'invention peut être préparée de la manière suivante, donnée à titre d'exemple.

La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, 'solvant de synthèse'.

Lorsque le milieu non aqueux choisi est une huile hydrocarbonée, ou siliconée, non volatile, on peut effectuer la polymérisation dans un solvant organique apolaire (solvant de synthèse) puis ajouter l'huile hydrocarbonée (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation.

En particulier, on peut choisir le solvant de synthèse parmi les alcanes tels que l'heptane ou le cyclohexane.

Lorsque le milieu non aqueux choisi est une huile hydrocarbonée volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y est insoluble.

Les monomères sont de préférence présents dans le solvant de synthèse, avant polymérisation, à raison de 5-20% en poids. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, lorsqu'elles se forment lors de la polymérisation, grâce à l'agent stabilisant.

La stabilisation peut être effectuée par tout moyen connu, et en particulier par ajout direct de l'agent stabilisant, lors de la polymérisation.

Le stabilisant est de préférence également présent dans le mélange avant polymérisation. Toutefois, il est également possible de l'ajouter en continu, notamment lorsque l'on ajoute également les monomères en continu.

On peut utiliser 2-30% en poids de stabilisant par rapport au mélange initial de monomères, et de préférence 5-20% en poids.

Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est silicone.

Comme copolymères blocs greffés ou séquencés comprenant au moins un bloc de type polyorganosiloxane et au moins d'un polyéther, on peut utiliser les copolyol diméthicones tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères d'acrylates ou de méthacrylates d'alcools en $C_1$-$C_4$, et d'acrylates ou de méthacrylates d'alcools en $C_8$-$C_{30}$, on peut utiliser le copolymère méthacrylate de stéaryle / méthacrylate de méthyle.

Comme copolymères blocs greffés ou séquencés comprenant au moins un bloc résultant de la polymérisation de diènes, hydrogéné ou non-hydrogéné, et au moins un bloc d'un polymère vinylique, on peut citer les copolymères séquencés, notamment de type 'dibloc' ou 'tribloc' du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation de diènes, hydrogéné ou non-hydrogéné, et au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation de diènes, hydrogéné ou non-hydrogéné, et au moins un bloc d'un polyéther, on peut citer les copolymères bi- ou triséquencés polyoxyéthylène/polybutadiène ou polyoxyéthylène/polyisobutylène.

Lorsque l'on utilise un polymère statistique en tant que stabilisant, on le choisit de manière à ce qu'il possède une quantité suffisante de groupements le rendant soluble dans le solvant de synthèse envisagé.

Les dispersion obtenues selon l'invention peuvent alors être utilisées dans une composition notamment cosmétique, pharmaceutique et/ou hygiénique, telle qu'une composition de soin ou de maquillage de la peau ou des matières kératiniques, ou encore une composition capillaire ou une composition solaire.

Suivant l'application, on pourra choisir d'utiliser des dispersion de polymères filmifiables ou non filmifiables, dans des huiles volatiles ou non volatiles.

La composition peut alors contenir, selon l'application envisagée, les constituants habituels à ce type de composition.

Parmi ces constituants, on peut citer les corps gras, et notamment les cires, les huiles, les gommes et/ou les corps gras pâteux, hydrocarbonés et/ou siliconés, et les composés pulvérulents tels que les pigments, les charges et/ou les nacres.

Parmi les cires susceptibles d'être présentes dans la composition selon l'invention, on peut citer, seules ou en mélange, les cires hydrocarbonées telles que la cire d'abeilles ; la cire de Carnauba, de Candellila, d'Ourrury, du Japon, les cires de fibres de lièges ou de canne à sucre ; les cires de paraffine, de lignite ; les cires microcristallines ; la cire de lanoline; la cire de montan ; les ozokérites ; les cires de polyéthylène ; les cires obtenues par synthèse de Fischer-Tropsch ; les huiles hydrogénés, les esters gras et les glycérides concrets à 25°C. On peut également utiliser des cires de silicone, parmi lesquelles on peut citer les alkyls, alcoxys et/ou esters de polyméthylsiloxane.

Parmi les huiles susceptibles d'être présentes dans la composition selon l'invention, on peut citer, seules ou en mélange, les huiles hydrocarbonées telles que l'huile de paraffine ou de vaseline ; le perhydrosqualène ; l'huile d'arara ; l'huile d'amande douce, de calophyllum, de palme, de ricin, d'avocat, de jojoba, d'olive ou de germes de céréales ; des esters d'acide lanolique, d'acide oléique, d'acide laurique, d'acide stéarique ; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

Les pigments peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Les nacres peuvent être choisies parmi le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères creuses telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingocéryls, des filtres solaires, des tensioactifs, des polymères liposolubles comme les polyalkylènes, notamment le polybutène, les polyacrylates et les polymères siliconés compatibles avec les corps gras. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Les compositions selon l'invention peuvent se présenter sous toute forme acceptable et usuelle pour une composition cosmétique, hygiénique ou pharmaceutique.

En particulier, la composition selon l'invention peut se présenter sous forme d'émulsion huile-dans-eau ou eau-dans-huile, de lotion, de mousse, de spray.

Parmi les applications préférentiellement visées par la présente invention, on peut plus particulièrement mentionner :

- le domaine des produits capillaires (lavage, soin ou beauté des cheveux), les compositions selon l'invention étant en particulier sous forme d'aérosols, de mousse, de shampooings, d'après-shampooings, de lotions ou de gels coiffants ou traitants, laques ou lotions de mise en forme ou de mise en plis ou encore de fixation. Dans ce cas, la composition capillaire comprend de préférence une dispersion de polymère réticulée dans une huile de silicone.
- le domaine des produits de maquillage, en particulier pour le maquillage des cils, les compositions étant sous forme de mascara ou de eye-liner ; de rouge à lèvres ou de brillant à lèvres ou de fond de teint.

L'invention est illustrée plus en détail dans les exemples suivants.

Exemple 1

On mélange 360 g de n-heptane et 15 g de polymère stabilisant séquencé de type copolymère dibloc polystyrène/copoly(éthylène-propylène) vendu sous la dénomination KRATON G1701 (Shell).
On chauffe le mélange pendant au moins 3 h, à environ 60°C afin d'obtenir une solution dispersée.
A 25°C, on ajoute au mélange 19 g de méthacrylate de méthyle, 1 g de diméthacrylate d'éthylène glycol, 0,4 g de tertiobutylperoxy-2-éthylhexanoate (Trigonox 21S de Akzo) et 5 g d'heptane.
On chauffe le mélange à 75°C, sous azote, pendant au moins 3 heures.
On ajoute ensuite, à 75°C et pendant 1,5 heure, un mélange de 76 g de méthacrylate de méthyle, 4 g de diméthacrylate d'éthylène glycol, 1,6 g de tertiobutylperoxy-2-éthylhexanoate (Trigonox 21S de Akzo) et 80 g d'heptane.
A la fin de l'ajout, on chauffe à 85°C pendant 4 heures, on ajoute 1 g de Trigonox dissous dans 5 g d'heptane, et on chauffe encore à 85°C pendant 7 heures.
On obtient une dispersion stable d'aspect laiteux, avec un taux de matière sèche de 18,6% en poids.
La mesure de la taille des particules, effectuée par diffusion quasi-élatique de la lumière avec un Coulter N4 SD, donne les résultats suivants :

.   taille moyenne des particules : 160 nm
.   polydispersité : inférieure à 0,1.

On mélange 50 g de la dispersion dans l'heptane ci-dessus avec 28,5 g d'huile de paraffine non volatile. On évapore sélectivement l'heptane à l'aide d'un évaporateur rotatif.
On obtient alors une dispersion stable d'aspect laiteux, ayant un taux de matière sèche de 25% en poids, de polyméthacrylate de méthyle réticulé par le diméthacrylate d'éthylène glycol, dans une huile de paraffine.

Exemple 2

On prépare une dispersion de polyméthacrylate de méthyle réticulé par le diméthacrylate d'éthylène glycol, dans une huile de paraffine ramifiée et volatile (ISOPAR L de Exxon), selon la méthode de l'exemple 1 en remplaçant l'heptane par ladite huile de paraffine ISOPAR L.
On obtient ainsi une dispersion stable, ayant un taux de matière sèche de 19% en poids et une taille moyenne des particules de 159 nm (polydispersité 0,05).

Exemple 3

On mélange 20 g de la dispersion dans l'ISOPAR ci-dessus avec 16,2 g de cyclotétradiméthylsiloxane (huile de silicone volatile).
On obtient alors une dispersion stable d'aspect laiteux, constituée de 3,8 g de polyméthacrylate de méthyle réticulé par le diméthacrylate d'éthylène glycol, 16,2 g d'huile de paraffine volatile et 16,2 g d'huile de silicone volatile.

Exemple 4

On mélange 20 g de la dispersion dans l'ISOPAR de l'exemple 2 avec 16,2 g de benzoate d'alcools en $C_{12}$-$C_{15}$ (FINSOLV TN de Witco).
On obtient alors une dispersion stable d'aspect laiteux, constituée de 3,8 g de polyméthacrylate de méthyle réticulé par le diméthacrylate d'éthylène glycol, 16,2 g d'huile de paraffine volatile et 16,2 g d'ester non volatil.

Exemple 5

On mélange 360 g de n-heptane et 15 g de polymère stabilisant séquencé de type copolymère dibloc polystyrène/copoly(éthylène-propylène) vendu sous la dénomination KRATON G1701 (Shell).

On chauffe le mélange pendant au moins 3 h, à environ 60°C afin d'obtenir une solution dispersée.

A 25°C, on ajoute au mélange 20 g d'acrylate de méthyle, 0,4 g de tertiobutylperoxy-2-éthylhexanoate et 5 g d'heptane. On chauffe le mélange à 75°C, sous azote, pendant 3 heures. On ajoute à 75°C et pendant 1,5 heures, un mélange de 80 g d'acrylate de méthyle, 1,6 g de tertiobutylperoxy-2-éthylhexanoate et 80 g d'heptane.

A la fin de l'ajout, on chauffe à 85°C pendant 4 heures, puis on ajoute 1 g de Trigonox dissous dans 5 g d'heptane, et on chauffe encore à 85°C pendant 7 heures.

On obtient une dispersion stable d'aspect laiteux, avec un taux de matière sèche de 19% en poids.

La mesure de la taille des particules, effectuée par diffusion quasi-élatique de la lumière avec un Coulter N4 SD, donne les résultats suivants :

. taille moyenne des particules ; 230 nm
. polydispersité : inférieure à 0,1.

On mélange 50 g de la dispersion dans l'heptane ci-dessus avec 28,5 g d'huile de paraffine non volatile. On évapore sélectivement l'heptane à l'aide d'un évaporateur rotatif.

On obtient alors une dispersion stable d'aspect laiteux, ayant un taux de matière sèche de 25% en poids, de polyacrylate de méthyle (Tg = 10°C) dans une huile de paraffine non volatile.

Exemple 6

On prépare une dispersion de polyacrylate de méthyle dans une huile de paraffine ramifiée et volatile (ISOPAR L de Exxon), de la même manière que dans l'exemple 5, en remplaçant l'heptane par ladite huile de paraffine ISOPAR L. On obtient ainsi une dispersion stable, ayant un taux de matière sèche de 20% en poids et une taille moyenne des particules de 197 nm (polydispersité 0,06).

Cette dispersion est filmogène et donne, après séchage, un film continu et transparent.

Exemple 7

On a mélangé 360 g d'isododécane et 15 g de polymère stabilisant séquencé de type copolymère dibloc polystyrène/copoly(éthylène-propylène) vendu sous la dénomination KRATON G1701 (Shell).

On a chauffé le mélange pendant au moins 3 heures, à environ 60°C afin d'obtenir une solution dispersée.

A 25°C, on a ajouté au mélange 48 g d'acrylate de méthyle, 5 g d'acrylate de butyle, 3 g d'acide acrylique, 0,4 g de tertiobutylperoxy-2-éthylhexanoate (Trigonox 21S de Akzo) et 5 g d'isododécane.

On a chauffé le mélange à 75°C, sous azote, pendant au moins 3 heures.

On a ajouté ensuite, à 75°C et pendant 1,5 heure, un mélange de 48 g d'acrylate de méthyle, 20 g d'acrylate de butyle, 12 g d'acide acrylique, 1,6 g de Trigonox 21S et 80 g d'isododécane.

A la fin de l'ajout, on a chauffé pendant 4 h à 85°C, puis on a ajouté 1 g de Trigonox 21S dissous dans 5 g d'isododécane et on a chauffé pendant 7 h à 85°C.

On a obtenu une dispersion stable avec un taux de matière sèche de 20,5 % en poids.

La mesure de la taille des particules, effectuée par diffusion quasi-élatique de la lumière avec un Coulter N4 SD, donne les résultats suivants :

. taille moyenne des particules : 175 nm
. polydispersité : inférieure à 0,1.

A 100 g de la dispersion ainsi obtenue, on a ajouté 3,2 g d'amino-2 méthyl-2 propanol puis laissé agiter pendant 1 heure. On a ensuite ajouté 2,7 g de glycérol puis laissé agiter pendant 5 h.

On a obtenu une dispersion stable et homogène. Le milieu de dispersion est limpide, montrant ainsi que le glycérol s'est incorporé dans les particules de polymère.

Exemple 8

On a mélangé 255 g de n-heptane et 150 g de diméthicone copolyol vendu sous la dénomination Dow Corning 3225 C par la société DOW CORNING (agent stabilisant siliconé).

On a chauffé le mélange à 60°C, puis on a ajouté 19 g de méthacrylate de méthyle, 1 g d'acide acrylique, 0,4 g de tertiobutylperoxy-2-éthylhexanoate (Trigonox 21S de Akzo) et 10 g d'heptane.

On a chauffé le mélange à 75°C, sous azote, pendant 3 h. Puis on a ajouté, à 75°C, pendant 1h30, le mélange de 76 g de méthacrylate de méthyle, 4 g d'acide méthacrylique, 1,6 g de Trigonox 21S et 50 g d'heptane. On a laissé réagir pendant 8 h à 75°C.

On a ainsi obtenu une dispersion stable à l'aspect laiteux, ayant un taux de matière sèche de 17,7 %.

La mesure de la taille des particules, effectuée par diffusion quasi-élatique de la lumière avec un Coulter N4 SD, donne les résultats suivants :

. taille moyenne des particules : 360 nm

. polydispersité : inférieure à 0,1.

Puis on a mélangé 50 g de la dispersion obtenue avec 30 g de diméthylcyclopentasiloxane puis on a évaporé sélectivement l'heptane. On a obtenu alors une dispersion stable.

Exemple 9

On a préparé une dispersion de polymère analogue à celle de l'exemple 8 en remplaçant le mélange de départ par un mélange constitué de 130 g d'heptane et de 17,7 g de lauryl méthicone copolyol vendu sous la dénomination "Dow Corning 5200" par la société DOW CORNING, à titre d'agent stabilisant.

On a ainsi obtenu une dispersion stable de particules.

La mesure de la taille des particules, effectuée par diffusion quasi-élatique de la lumière avec un Coulter N4 SD, donne les résultats suivants :

. taille moyenne des particules : 350 nm

. polydispersité: inférieure à 0,1.

Exemple 10: Crème antirides

On a préparé une émulsion huile-dans-eau ayant la composition suivante ;

Phase huileuse

| - Tristéarate de sorbitane | 0,9 g |
|---|---|
| - Monostérate de glycéryle | 3,0 g |
| - Huile d'abricot | 2,0 g |
| - Polyisobutylène hydrogéné | 4,0 g |
| - Stéarate de polyéthylèneglycol à 40 moles d'oxyde d'éthylène | 2,0 g |
| - Dispersion de l'exemple 2 | 7,0 g |

Phase aqueuse

| - Polyacrylamide en émulsion inverse à 40 % de MA (SEPIGEL 305 de la société SEPPIC) | 0,9 g |
|---|---|
| - Agent séquestrant | 0,05 g |
| - Conservateur | 0,5 g |
| - Glycérine | 3,0 g |
| - Eau          qsp | 100 g |

On a dispersé la phase huileuse dans la phase aqueuse sous agitation à l'homogénéisateur. On a ainsi obtenu une émulsion huile-dans-eau qui est utilisée comme crème antirides.

Exemple 11:

On prépare une émulsion ayant la composition suivante :

Phase huileuse

| | |
|---|---|
| - Diisostéarate de diglycéryle | 2,0 g |
| - Laurylméthicone copolyol (DOW CORNING Q2-5200) | 2,0 g |
| - Huile de vaseline | 1,0g |
| - Cyclométhicone | 21 g |
| - Dispersion de l'exemple 1 | 4, 0 g |

Phase aqueuse

| | | |
|---|---|---|
| - Sulfate de magnésium | | 1,0 g |
| - MéThyl parabène | | 0,25 g |
| - Chlorphenesine | | 0,3 g |
| - Eau | qsp | 100 g |

On procède selon le même mode opératoire qu'à l'exemple 1.

Exemple 12: Emulsion eau-dans-huile

On prépare une émulsion ayant la composition suivante :

Phase A

| | |
|---|---|
| - Acide stéarique | 0,4 g |
| - Stéarate de polyéthylèneglycol à 40 moles d'oxyde d'éthylène | 3,5 g |
| - Alcool cétylique | 3,2 g |
| - Mélange de mono, di et tristéarate de glycéryle | 3,0 g |
| - Myristate de myristyle | 2,0 g |
| - Myristate d'isopropyle | 7,0 g |
| - Dispersion de l'exemple 5 | 8,0 g |

Phase B

| - Cyclopentadiméthylsiloxane | 5,0 g |
|---|---|

Phase C

| - Glycérol | 3,0 g |
|---|---|
| - Conservateur | 0,2 g |
| - Eau          qsp | 100 g |

Les constituants de la phase A sont solubilisés à 80 °C. Lorsque le mélange est limpide, la température est abaissée à 65 °C et on ajoute la phase B. La température est maintenue à 65 °C.

Les constituants de la phase C sont solubilisés à 85°C-90°C, puis la température est ramenée à 65°C. On verse le mélange des phases A et B dans la phase C et on refroidit sous agitation jusqu'à la température ambiante.

On obtient une crème blanche de soin pour protéger quotidiennement la peau des méfaits du rayonnement UV et pour empêcher la formation des rides et ridules.

Exemple 13

On prépare un gel pour le visage ayant la composition suivante:

| . isopropyl palmitate | 10 g |
|---|---|
| . vaseline (cire) | 5 g |
| . hectorite modifiée (argile) | 0,15 g |
| . ozokérite (cire) | 5 g |
| . septaoléate de sorbitane oxyéthyléné (40OE) | 5 g |
| . dispersion de l'exemple 6 (25% de matière sèche) | 75 g |

On obtient un gel ayant de bonnes propriétés cosmétiques.

Exemple 14

On prépare une huile de soin ayant la composition suivante :

| . dispersion de l'exemple 4 | 70 g |
|---|---|
| . huile de jojoba | 15 g |
| . huile de soja | 15 g |

On obtient une huile de soin qui peut être appliquée sur le corps ou le visage.

Exemple 15

On a préparé une composition de fixation des cheveux selon le mode opératoire suivant :

A 50 g de la dispersion obtenue à l'exemple 1, on a ajouté 50 g de la dispersion de l'exemple 5 et 7,5 g d'huile de paraffine non volatile.

On a introduit ce mélange dans un flacon pompe.

Le produit a été pulvérisé sous forme de gouttelettes sur les cheveux. Après séchage, le produit présente une bonne adhésion sur les cheveux et occasionne des soudures rigides apportant le maintien de la coiffure.

Le produit est facilement éliminable par brossage.

**Revendications**

1. Composition comprenant, dans un milieu cosmétiquement, pharmaceutiquement et/ou hygiéniquement acceptable, une dispersion de particules d'au moins un polymère stabilisé en surface par un agent stabilisant dans un milieu non aqueux, caractérisé par le fait que :

   a) le milieu non aqueux est constitué d'au moins un composé liquide non aqueux choisi dans le groupe constitué par :

   - les composés liquides non aqueux ayant un paramètre de solubilité global selon l'espace de solubilité de HANSEN inférieur à 17 $(MPa)^{1/2}$,
   - ou les monoalcools ayant un paramètre de solubilité global selon l'espace de solubilité de HANSEN inférieur ou égal à 20 $(MPa)^{1/2}$,
   - ou leurs mélanges,

   et
   b)

   - (i) lorsque le milieu non aqueux comprend au moins une huile de silicone, l'agent stabilisant est choisi dans le groupe constitué par les copolymères blocs greffés ou séquencés comprenant au moins un bloc de type polyorganosiloxane et au moins un bloc d'un polymère radicalaire ou d'un polyéther ou d'un polyester,
   - (ii) lorsque le milieu non aqueux ne comprend pas une huile de silicone, l'agent stabilisant est choisi dans le groupe constitué par :

     - (a) les copolymères blocs greffés ou séquencés comprenant au moins un bloc de type polyorganosiloxane et au moins un bloc d'un polymère radicalaire ou d'un polyéther ou d'un polyester,
     - (b) les copolymères d'acrylates ou de méthacrylates d'alcools en $C_1$-$C_4$, et d'acrylates ou de méthacrylates d'alcools en $C_8$-$C_{30}$,
     - (c) les copolymères blocs greffés ou séquencés comprenant au moins un bloc résultant de la polymérisation de diènes, hydrogéné ou non-hydrogéné, et au moins un bloc d'un polymère vinylique ou acrylique ou d'un polyéther ou d'un polyester, ou leurs mélanges.

2. Composition selon la revendication 1, caractérisée par le fait que les particules de polymères stabilisées en surface sont des particules sphériques et ont une taille moyenne de 5 à 600 nm.

3. Composition selon l'une quelconque des revendications précédentes, caractérisée par le fait que le polymère stabilisé en surface est choisi parmi les polymères radicalaires, les polycondensats, et/ou les polymères d'origine naturelle.

4. Composition selon la revendication 3, caractérisée par le fait que le polymère stabilisé est réticulé.

5. Composition selon l'une quelconque des revendications précédentes, caractérisée par le fait que le polymère stabilisé est choisi parmi les homopolymères ou les copolymères acryliques ou vinyliques, éventuellement réticulés.

6. Composition selon l'une quelconque des revendications précédentes, caractérisée par le fait que le polymère stabilisé est choisi parmi le polyméthacrylate de méthyle, le polystyrène ou le polyacrylate de tertiobutyle.

7. Composition selon l'une des revendications précédentes, caractérisée par le fait que la dispersion comprend en outre un plastifiant.

8. Composition selon l'une quelconque des revendications précédentes, caractérisée par le fait que les monoalcools ayant un paramètre de solubilité global selon l'espace de solubilité de HANSEN inférieur ou égal à 20 (Mpa)$^{1/2}$ sont choisis dans le groupe formé par les monoalcools aliphatiques gras ayant au moins 6 atomes de carbones.

9. Composition selon l'une quelconque des revendications 1 à 7, caractérisée par le fait que le composé liquide non aqueux ayant un paramètre de solubilité global selon l'espace de solubilité de HANSEN inférieur à 17 (MPa)$^{1/2}$ est choisi parmi les huiles naturelles ou synthétiques, carbonées, hydrocarbonées, fluorées et/ou siliconées, seules ou en mélange ; les esters ; les cétones ; les éthers ; les alcanes linéaires, ramifiés et/ou cycliques, éventuellement volatils.

10. Composition selon l'une quelconque des revendications précédentes, caractérisée par le fait que le composé liquide non aqueux ayant un paramètre de solubilité global selon l'espace de solubilité de HANSEN inférieur à 17 (MPa)$^{1/2}$ est choisi dans le groupe constitué par les esters linéaires, ramifiés ou cycliques, ayant plus de 6 atomes de carbone, les éthers ayant plus de 6 atomes de carbone, les cétones ayant plus de 6 atomes de carbone.

11. Composition selon l'une quelconque des revendications précédentes, caractérisée par le fait que le copolymère stabilisant séquencé comprenant au moins un bloc résultant de la polymérisation de diènes est choisi dans le groupe formé par les copolymères séquencés, polystyrène/polyisoprène, polystyrène/polybutadiène, polystyrène/copoly(éthylène-propylène) ou polystyrène/copoly(éthylène-butylène).

12. Composition selon l'une quelconque des revendications 1 à 10, caractérisée par le fait que le polymère stabilisant greffé comprenant au moins un bloc de type polyorganosiloxane et au moins un bloc copolymère radicalaire est choisi dans le groupe formé par les copolymères greffés de type acrylique/silicone.

13. Composition selon l'une quelconque des revendications 1 à 10, caractérisée par le fait que le polymère stabilisant greffé comprenant au moins un bloc de type polyorganosiloxane et au moins d'un polyéther est choisi dans le groupe formé par les copolyols diméthicone et les lauryl diméthicones.

14. Composition selon l'une quelconque des revendications 1 à 10, caractérisée par le fait que le copolymère bloc stabilisant, greffé ou séquencé comprenant au moins un bloc résultant de la polymérisation de diènes, hydrogéné ou non-hydrogéné, et au moins un bloc d'un polymère acrylique, est choisi dans le groupe constitué par les copolymères bi- ou triséquencés poly (méthylacrylate de méthyle)/polyisobutylène ou les copolymères greffés à squelette poly(méthylacrylate de méthyle) et à greffons polyisobutylène.

15. Composition selon l'une quelconque des revendications 1 à 10, caractérisée par le fait que le copolymère bloc stabilisant, greffé ou séquencé comprenant au moins un bloc résultant de la polymérisation de diènes, hydrogéné ou non-hydrogéné, et au moins un bloc d'un polyéther, est choisi dans le groupe constitué par les copolymères bi- ou triséquencés polyoxyéthylène/ polybutadiène ou polyoxyéthylène/polyisobutylène.

16. Composition selon l'une quelconque des revendications précédentes, caractérisée par le fait que le stabilisant est présent dans la dispersion à raison de 2-30% en poids de stabilisant par rapport au mélange initial de monomères servant à former le polymère à stabiliser, et de préférence 5-20% en poids.

17. Composition selon l'une des revendications précédentes, caractérisée par le fait qu'elle comprend en outre des corps gras choisis dans le groupe formé par les cires, les huiles, les gommes, les corps gras pâteux, hydrocarbonés ou silicones.

18. Composition selon l'une des revendications précédentes, caractérisée par le fait qu'elle comprend en outre des pigments, des charges et/ou des nacres.

19. Composition selon l'une des revendications précédentes, caractérisée par le fait qu'elle se présente sous la forme d'une composition de soin ou de maquillage de la peau ou des matières kératiniques, ou encore d'une composition capillaire ou d'une composition solaire.

20. Utilisation d'une dispersion de particules d'au moins un polymère stabilisé en surface par un agent stabilisant dans un milieu non aqueux, dans et pour la préparation d'une composition cosmétique, hygiénique ou pharmaceutique, caractérisée par le fait que :

a) le milieu non aqueux est constitué d'au moins un composé liquide non aqueux choisi dans le groupe constitué par :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges,

et
b)

- (i) lorsque le milieu non aqueux comprend au moins une huile de silicone, l'agent stabilisant est choisi dans le groupe constitué par les copolymères blocs greffés ou séquencés comprenant au moins un bloc de type polyorganosiloxane et au moins un bloc d'un polymère radicalaire ou d'un polyéther ou d'un polyester,
- (ii) lorsque le milieu non aqueux ne comprend pas une huile de silicone, l'agent stabilisant est choisi dans le groupe constitué par:

  - (a) les copolymères blocs greffés ou séquencés comprenant au moins un bloc de type polyorganosiloxane et au moins un bloc d'un polymère radicalaire ou d'un polyéther ou d'un polyester,
  - (b) les copolymères d'acrylates ou de méthacrylates d'alcools en $C_1$-$C_4$, et d'acrylates ou de méthacrylates d'alcools en $C_8$-$C_{30}$,
  - (c) les copolymères blocs greffés ou séquencés comprenant au moins un bloc résultant de la polymérisation de diènes, hydrogéné ou non-hydrogéné, et au moins un bloc d'un polymère vinylique ou acrylique ou d'un polyéther ou d'un polyester, ou leurs mélanges.

21. Utilisation d'une dispersion de particules d'au moins un polymère non filmogène stabilisé en surface par un agent stabilisant dans un milieu non aqueux

a) le milieu non aqueux étant constitué d'au moins un composé liquide non aqueux choisi dans le groupe constitué par:

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges,

et
b)

- (i) lorsque le milieu non aqueux comprend au moins une huile de silicone, l'agent stabilisant est choisi dans le groupe constitué par les copolymères blocs greffés ou séquencés comprenant au moins un bloc de type polyorganosiloxane et au moins un bloc d'un polymère radicalaire ou d'un polyéther ou d'un polyester,
- (ii) lorsque le milieu non aqueux ne comprend pas une huile de silicone, l'agent stabilisant est choisi dans le groupe constitué par:

  - (a) les copolymères blocs greffés ou séquencés comprenant au moins un bloc de type polyorganosiloxane et au moins un bloc d'un polymère radicalaire ou d'un polyéther ou d'un polyester,
  - (b) les copolymères d'acrylates ou de méthacrylates d'alcools en C1-C4, et d'acrylates ou de méthacrylates d'alcools en $C_8$-$C_{30}$,
  - (c) les copolymères blocs greffés ou séquencés comprenant au moins un bloc résultant de la polymérisation de diènes, hydrogéné ou non-hydrogéné, et au moins un bloc d'un polymère vinylique ou acrylique ou d'un polyéther ou d'un polyester, ou leurs mélanges,

comme charge dans une composition cosmétique, hygiénique ou pharmaceutique.

## Claims

1. Composition comprising, in a cosmetically, pharmaceutically and/or hygienically acceptable medium, a dispersion of particles of at least one polymer stabilized at the surface by a stabilizer in a non-aqueous medium, characterized in that:

   a) the non-aqueous medium consists of at least one non-aqueous liquid compound chosen from the group consisting of:

   - non-aqueous liquid compounds having a global solubility parameter, according to the Hansen solubility space, of less than 17 $(MPa)^{1/2}$,
   - or monoalcohols having a global solubility parameter, according to the Hansen solubility space, of less than or equal to 20 $(MPa)^{1/2}$,
   - or mixtures thereof,

   and
   b)

   - (i) when the non-aqueous medium comprises at least one silicone oil, the stabilizer is chosen from the group consisting of sequential or grafted block copolymers comprising at least one block of polyorganosiloxane type and at least one block of a radical polymer or of a polyether or of a polyester,
   - (ii) when the non-aqueous medium does not comprise a silicone oil, the stabilizer is chosen from the group consisting of:

     - (a) sequential or grafted block copolymers comprising at least one block of polyorganosiloxane type and at least one block of a radical polymer or of a polyether or of a polyester,
     - (b) copolymers of acrylates or methacrylates of $C_1$-$C_4$ alcohols, and of acrylates or methacrylates of $C_8$-$C_{30}$ alcohols,
     - (c) sequential or grafted block copolymers comprising at least one block resulting from the polymerization of dienes, which is hydrogenated or non-hydrogenated, and at least one block of a vinyl or acrylic polymer or of a polyether or of a polyester, or mixtures thereof.

2. Composition according to Claim 1, characterized in that the surface-stabilized polymer particles are spherical particles and have an average diameter of from 5 to 600 nm.

3. Composition according to either of the preceding claims, characterized in that the surface-stabilized polymer is chosen from radical polymers, polycondensates and/or polymers of natural origin.

4. Composition according to Claim 3, characterized in that the stabilized polymer is crosslinked.

5. Composition according to any one of the preceding claims, characterized in that the stabilized polymer is chosen from acrylic or vinyl copolymers or homopolymers, which are optionally crosslinked.

6. Composition according to any one of the preceding claims, characterized in that the stabilized polymer is chosen from polymethyl methacrylate, polystyrene and poly-tert-butyl acrylate.

7. Composition according to one of the preceding claims, characterized in that the dispersion also comprises a plasticizer.

8. Composition according to any one of the preceding claims, characterized in that the monoalcohols having a global solubility parameter according to the Hansen solubility space of less than or equal to 20 $(MPa)^{1/2}$ are chosen from the group formed by fatty aliphatic monoalcohols having at least 6 carbon atoms.

9. Composition according to any one of Claims 1 to 7, characterized in that the non-aqueous liquid compound having a global solubility parameter according to the Hansen solubility space of less than 17 $(MPa)^{1/2}$ is chosen from natural or synthetic, carbon-based, hydrocarbon, fluoro and/or silicone oils, alone or as a mixture; esters; ketones; ethers; linear, branched and/or cyclic alkanes, which are possibly volatile.

10. Composition according to any one of the preceding claims, characterized in that the non-aqueous liquid compound having a global solubility parameter according to the Hansen solubility space of less than 17 $(MPa)^{1/2}$ is chosen from the group consisting of linear, branched or cyclic esters having more than 6 carbon atoms, ethers having more than 6 carbon atoms and ketones having more than 6 carbon atoms.

11. Composition according to any one of the preceding claims, characterized in that the sequential stabilizing copolymer comprising at least one block resulting from the polymerization of dienes is chosen from the group formed by polystyrene/polyisoprene, polystyrene/polybutadiene, polystyrene/copoly(ethylene-propylene) and polystyrene/copoly(ethylene-butylene) sequential copolymers.

12. Composition according to any one of Claims 1 to 10, characterized in that the grafted stabilizing polymer comprising at least one block of polyorganosiloxane type and at least one radical copolymer block is chosen from the group formed by grafted copolymers of acrylic/silicone type.

13. Composition according to any one of Claims 1 to 10, characterized in that the grafted stabilizing polymer comprising at least one block of polyorganosiloxane type and at least one polyether is chosen from the group formed by dimethicone copolyols and lauryl dimethicones.

14. Composition according to any one of Claims 1 to 10, characterized in that the sequential or grafted stabilizing block copolymer comprising at least one block resulting from the polymerization of dienes, which is hydrogenated or non-hydrogenated, and at least one block of an acrylic polymer, is chosen from the group consisting of poly(methyl methacrylate)/polyisobutylene di- or trisequential copolymers or grafted copolymers containing a poly(methyl methacrylate) skeleton and containing polyisobutylene grafts.

15. Composition according to any one of Claims 1 to 10, characterized in that the sequential or grafted stabilizing block copolymer comprising at least one block resulting from the polymerization of dienes, which is hydrogenated or non-hydrogenated, and at least one block of a polyether, is chosen from the group consisting of polyoxyethylene/polybutadiene or polyoxyethylene/polyisobutylene di- or trisequential copolymers.

16. Composition according to any one of the preceding claims, characterized in that the stabilizer is present in the dispersion in a proportion of 2-30 % by weight of stabilizer relative to the initial monomer mixture which serves to form the polymer to be stabilized, and preferably 5-20 % by weight.

17. Composition according to one of the preceding claims, characterized in that it also comprises fatty substances chosen from the group formed by waxes, oils, gums and pasty fatty substances, which are hydrocarbon-based or silicone-based.

18. Composition according to one of the preceding claims, characterized in that it also comprises pigments, fillers and/or pearlescent agents.

19. Composition according to one of the preceding claims, characterized in that it is in the form of a composition to care for or make up the skin or keratin substances, or alternatively a hair composition or a sun composition.

20. Use of a dispersion of particles of at least one polymer stabilized at the surface by a stabilizer in a non-aqueous medium, in and for the preparation of a cosmetic, hygiene or pharmaceutical composition, characterized in that:

   a) the non-aqueous medium consists of at least one non-aqueous liquid compound chosen from the group consisting of:

   - non-aqueous liquid compounds having a global solubility parameter, according to the Hansen solubility space, of less than 17 $(MPa)^{1/2}$,
   - or monoalcohols having a global solubility parameter, according to the Hansen solubility space, of less than or equal to 20 $(MPa)^{1/2}$,
   - or mixtures thereof,

and

b)

- (i) when the non-aqueous medium comprises at least one silicone oil, the stabilizer is chosen from the group consisting of sequential or grafted block copolymers comprising at least one block of polyorganosiloxane type and at least one block of a radical polymer or of a polyether or of a polyester,
- (ii) when the non-aqueous medium does not comprise a silicone oil, the stabilizer is chosen from the group consisting of:

    - (a) sequential or grafted block copolymers comprising at least one block of polyorganosiloxane type and at least one block of a radical polymer or of a polyether or of a polyester,
    - (b) copolymers of acrylates or methacrylates of $C_1$-$C_4$ alcohols, and of acrylates or methacrylates of $C_8$-$C_{30}$ alcohols,
    - (c) sequential or grafted block copolymers comprising at least one block resulting from the polymerization of dienes, which is hydrogenated or non-hydrogenated, and at least one block of a vinyl or acrylic polymer or of a polyether or of a polyester, or mixtures thereof.

21. Use of a dispersion of particles of at least one non-film-forming polymer stabilized at the surface by a stabilizer in a non-aqueous medium

    a) the non-aqueous medium consisting of at least one non-aqueous liquid compound chosen from the group consisting of:

    - non-aqueous liquid compounds having a global solubility parameter, according to the Hansen solubility space, of less than 17 $(MPa)^{1/2}$,
    - or monoalcohols having a global solubility parameter, according to the Hansen solubility space, of less than or equal to 20 $(MPa)^{1/2}$,
    - or mixtures thereof,

    and

    b)

    - (i) when the non-aqueous medium comprises at least one silicone oil, the stabilizer is chosen from the group consisting of sequential or grafted block copolymers comprising at least one block of polyorganosiloxane type and at least one block of a radical polymer or of a polyether or of a polyester,
    - (ii) when the non-aqueous medium does not comprise a silicone oil, the stabilizer is chosen from the group consisting of:

        - (a) sequential or grafted block copolymers comprising at least one block of polyorganosiloxane type and at least one block of a radical polymer or of a polyether or of a polyester,
        - (b) copolymers of acrylates or methacrylates of $C_1$-$C_4$ alcohols, and of acrylates or methacrylates of $C_8$-$C_{30}$ alcohols,
        - (c) sequential or grafted block copolymers comprising at least one block resulting from the polymerization of dienes, which is hydrogenated or non-hydrogenated, and at least one block of a vinyl or acrylic polymer or of a polyether or of a polyester, or mixtures thereof, as a filler in a cosmetic, hygiene or pharmaceutical composition.

**Patentansprüche**

1. Zusammensetzungen, die in einem kosmetisch, pharmazeutisch und/oder hygienisch akzeptablen Medium eine Dispersion von Partikeln mindestens eines an der Oberfläche durch ein Stabilisierungsmittel stabilisierten Polymers in einem nicht wäßrigen Medium enthalten, dadurch gekennzeichnet, daß:

    a) das nicht wäßrige Medium aus mindestens einer flüssigen, nicht wäßrigen Verbindung besteht, die ausgewählt ist unter:

    - den nicht wäßrigen, flüssigen Verbindungen mit einem Gesamtsolubilitätsparameter nach dem Solubilitätsraum von HANSEN unter 17 $(MPa)^{1/2}$, oder

- den Monoalkoholen mit einem Gesamtsolubilitätsparameter nach dem Solubilitätsraum von HANSEN von höchstens 20 (MPa)$^{1/2}$, oder
- deren Gemischen, und

b)

- (i) wenn das nicht wäßrige Medium mindestens ein Siliconöl enthält, das Stabilisierungsmittel unter den gepfropften oder sequentiellen Blockcopolymeren ausgewählt ist, die mindestens einen Block vom Polyorganosiloxantyp und mindestens einen Block eines durch radikalische Polymerisation hergestellten Polymers oder eine Polyethers oder eines Polyesters enthalten,
- (ii) wenn das nicht wäßrige Medium kein Siliconöl enthält, das Stabilisierungsmittel ausgewählt ist unter:

    - (a) den gepfropften oder sequentiellen Blockcopolymeren, die mindestens einen Block vom Polyorganosiloxantyp und mindestens einem Block eines durch radikalische Polymerisation hergestellten Polymers oder eines Polyethers oder eines Polyesters enthalten,
    - (b) den Copolymeren von Acrylaten oder Methacrylaten von $C_{1-4}$-Alkoholen und Acrylaten oder Methacrylaten von $C_{8-30}$-Alkoholen,
    - (c) den gepfropften oder sequentiellen Blockcopolymeren, die mindestens einen aus der Polymerisation von Dienen resultierenden, hydrierten oder nicht hydrierten Block und mindestens einen Block eines Vinyl- oder Acrylpolymers, eines Polyethers oder eines Polyesters enthalten oder deren Gemischen.

2. Zusammensetzungen nach Anspruch 1, dadurch gekennzeichnet, daß die an der Oberfläche stabilisierten Polymerpartikel kugelförmige Partikel sind und eine mittlere Größe von 5 bis 600 nm aufweisen.

3. Zusammensetzungen nach einem der vorhergehenden Ansprüche, dadurch gekennzeichnet, daß das an der Oberfläche stabilisierte Polymer unter den durch radikalische Polymerisation hergestellten Polymeren, den Polykondensaten und/oder Polymeren natürlichen Ursprungs ausgewählt sind.

4. Zusammensetzungen nach Anspruch 3, dadurch gekennzeichnet, daß das stabilisierte Polymer vernetzt ist.

5. Zusammensetzungen nach einem der vorhergehenden Ansprüche, dadurch gekennzeichnet, daß das stabilisierte Polymer unter den Acryl- oder Vinylhomopolymeren oder Acryl- oder Vinylcopolymeren, die gegebenenfalls vernetzt sind, ausgewählt sind.

6. Zusammensetzungen nach einem der vorhergehenden Ansprüche, dadurch gekennzeichnet, daß das stabilisierte Polymer unter Polymethylmethacrylat, Polystyrol oder Poly-tert.-butylacrylat ausgewählt ist.

7. Zusammensetzungen nach einem der vorhergehenden Ansprüche, dadurch gekennzeichnet, daß die Dispersion ferner einen Weichmacher enthält.

8. Zusammensetzungen nach einem der vorhergehenden Ansprüche, dadurch gekennzeichnet, daß die Monoalkohole, die einen Gesamtsolubilitätsparameter nach dem Solubilitätsraum von HANSEN von höchstens 20 (MPa)$^{1/2}$ aufweisen, unter den aliphatischen, einwertigen Fettalkoholen mit mindestens 6 Kohlenstoffatomen ausgewählt sind.

9. Zusammensetzungen nach einem der Ansprüche 1 bis 7, dadurch gekennzeichnet, daß die nicht wäßrige flüssige Verbindung, die einen Gesamtsolubilitätsparameter nach dem Solubilitätsraum von HANSEN unter 17 (MPa)$^{1/2}$ aufweist, unter den natürlichen oder synthetischen Ölen, Ölen auf Kohlenstoffbasis, Kohlenwasserstoffölen, fluorierten Ölen und/oder Siliconölen oder deren Gemischen, den Estern, Ketonen, Ethern und den geradkettigen, verzweigten und/oder cyclischen Alkanen, die gegebenenfalls flüchtig sind, ausgewählt ist.

10. Zusammensetzungen nach einem der vorhergehenden Ansprüche, dadurch gekennzeichnet, daß die nicht wäßrige, flüssige Verbindung, die einen Gesamtsolubilitätsparameter nach dem Solubilitätsraum von HANSEN unter 17 (MPa)$^{1/2}$ aufweist, unter den geradkettigen, verzweigten oder cyclischen Estern mit mehr als 6 Kohlenstoffatomen, Ethern mit mehr als 6 Kohlenstoffatomen oder Ketonen mit mehr als 6 Kohlenstoffatomen ausgewählt ist.

11. Zusammensetzungen nach einem der vorhergehenden Ansprüche, dadurch gekennzeichnet, daß das stabilisierende Blockcopolymer, das mindestens einen aus der Polymerisation von Dienen resultierenden Block aufweist, unter den sequentiellen Copolymeren Polystyrol/Polyisopren, Polystyrol/Polybutadien, Polystyrol/Copoly(ethylenpropylen) oder Polystyrol/Copoly(ethylen-butylen) ausgewahlt ist.

12. Zusammensetzungen nach einem der Ansprüche 1 bis 10, dadurch gekennzeichnet, daß das gepfropfte, stabilisierende Polymer, das mindestens einen Block vom Polyorganosiloxantyp und mindestens einen Block eines durch radikalische Polymerisation hergestellten Copolymers enthält, unter den gepfropften Copolymeren vom Typ Acryl/Silicon ausgewählt ist.

13. Zusammensetzungen nach einem der Ansprüche 1 bis 10, dadurch gekennzeichnet, daß das gepfropfte, stabilisierende Polymer, das mindestens einen Block vom Typ Polyorganosiloxan und mindestens einen Polyetherblock enthält, unter den Dimethicon-Copolyolen und Lauryldimethiconen ausgewählt ist.

14. Zusammensetzungen nach einem der Ansprüche 1 bis 10, dadurch gekennzeichnet, daß das gepfropfte oder sequentielle, stabilisierende Blockcopolymer, das mindestens einen sich bei der Polymerisation von Dienen ergebenden, hydrierten oder nicht hydrierten Block und mindestens einen Block eines Acrylpolymers enthält, unter den Zwei- oder Dreiblock-Copolymeren Poly(methylmethacrylat)/Polyisobutylen oder den gepfropften Copolymeren mit Polymethylmethacrylat-Gerüst und Polyisobutylen-Pfropfzweigen ausgewählt ist.

15. Zusammensetzungen nach einem der Ansprüche 1 bis 10, dadurch gekennzeichnet, daß das gepfropfte oder sequentielle, stabilisierende Blockcopolymer, das mindestens einen sich bei der Polymerisation von Dienen ergebenden hydrierten oder nicht hydrierten Block und mindestens einem Polyetherblock aufweist, unter den Zwei- oder Dreiblock-Copolymeren Polyoxyethylen/Polybutadien oder Polyoxyethylen/Polyisobutylen ausgewählt ist.

16. Zusammensetzungen nach einem der vorhergehenden Ansprüche, dadurch gekennzeichnet, daß das stabilisierungsmittel in der Dispersion in einem Mengenanteil von 2 bis 30 Gew.-% Stabilisierungsmittel, bezogen auf das anfänglich vorliegende Monomerengemisch, das das zu stabilisierende Polymer bilden soll, und vorzugsweise von 5 bis 20 Gew.-% vorliegt.

17. Zusammensetzungen nach einem der vorhergehenden Ansprüche, dadurch gekennzeichnet, daß sie ferner Fettsubstanzen enthalten, die unter den Wachsen, Ölen, Gummen, pastösen Fettsubstanzen, Kohlenwasserstoffen oder Siliconen ausgewählt ist.

18. Zusammensetzungen nach einem der vorhergehenden Ansprüche, dadurch gekennzeichnet, daß sie ferner Pigmente, Füllstoffe und/oder Perlglanzpigmente enthalten.

19. Zusammensetzungen nach einem der vorhergehenden Ansprüche, dadurch gekennzeichnet, daß sie in Form einer Zusammensetzung zur Pflege oder zum Schminken der Haut oder zur Pflege oder zum Schminken von Keratinmaterialien oder auch als Zusammensetzung für das Haar oder als Sonnenschutzmittel vorliegen.

20. Verwendung einer Dispersion von Partikeln mindestens eines an der Oberfläche durch ein Stabilisierungsmittel stabilisiertes Polymer in einem nicht wäßrigen Medium in einer kosmetischen, hygienischen oder pharmazeutischen Zusammensetzung oder zu deren Herstellung, dadurch gekennzeichnet, daß

a) das nicht wäßrige Medium aus mindestens einer flüssigen, nicht wäßrigen Verbindung besteht, die ausgewählt ist unter:

- den nicht wäßrigen, flüssigen Verbindungen mit einem Gesamtsolubilitätsparameter nach dem Solubilitätsraum von HANSEN unter 17 $(MPa)^{1/2}$, oder
- den Monoalkoholen mit einem Gesamtsolubilitätsparameter nach dem Solubilitätsraum von HANSEN von höchstens 20 $(MPa)^{1/2}$, oder
- deren Gemischen, und

b)

- (i) wenn das nicht wäßrige Medium mindestens ein Siliconöl enthält das Stabilisierungsmittel unter den gepfropften oder sequentiellen Blockcopolymeren ausgewählt ist, die mindestens einen Block vom Polyorganosiloxantyp und mindestens einen Block eines durch radikalische Polymerisation hergestellten Polymers oder eine Polyethers oder eines Polyesters enthalten,

- (ii) wenn das nicht wäßrige Medium kein Siliconöl enthält, das Stabilisierungsmittel ausgewählt ist unter:

  - (a) den gepfropften oder sequentiellen Blockcopolymeren, die mindestens einen Block vom Polyorganosiloxantyp und mindestens einem Block eines durch radikalische Polymerisation hergestellten Polymers oder eines Polyethers oder eines Polyesters enthalten,
  - (b) den Copolymeren von Acrylaten oder Methacrylaten von $C_{1-4}$-Alkoholen und Acrylaten oder Methacrylaten von $C_{8-30}$-Alkoholen,
  - (c) den gepfropften oder sequentiellen Blockcopolymeren, die mindestens einen sich bei der Polymerisation von Dienen ergebenden, hydrierten oder nicht hydrierten Block und mindestens einen Block eines Vinyl- oder Acrylpolymers oder eines Polyethers oder eines Polyesters aufweusen, oder deren Gemischen.

21. Verwendung einer Dispersion von Partikeln mindestens eines nicht filmbildenden an der Oberfläche durch ein Stabilisierungsmittel stabilisierten Polymers in einem nicht wäßrigen Medium

   a) das nicht wäßrige Medium aus mindestens einer flüssigen, nicht wäßrigen Verbindung besteht, die ausgewählt ist unter:

   - den nicht wäßrigen, flüssigen Verbindungen mit einem Gesamtsolubilitätsparameter nach dem Solubilitätsraum von HANSEN unter 17 $(MPa)^{1/2}$, oder
   - den Monoalkoholen mit einem Gesamtsolubilitätsparameter nach dem Solubilitätsraum von HANSEN von höchstens 20 $(MPa)^{1/2}$, oder
   - deren Gemischen, und

   b)

   - (i) wenn das nicht wäßrige Medium mindestens ein Siliconöl enthält, das Stabilisierungsmittel unter den gepfropften oder sequentiellen Blockcopolymeren ausgewählt ist, die mindestens einen Block vom Polyorganosiloxantyp und mindestens einen Block eines durch radikalische Polymerisation hergestellten Polymers oder eine Polyethers oder eines Polyesters enthalten,
   - (ii) wenn das nicht wäßrige Medium kein Siliconöl enthält, das Stabilisierungsmittel ausgewählt ist unter:

     - (a) den gepfropften oder sequentiellen Blockcopolymeren, die mindestens einen Block vom Polyorganosiloxantyp und mindestens einem Block eines durch radikalische Polymerisation hergestellten Polymers oder eines Polyethers oder eines Polyesters enthalten,
     - (b) den Copolymeren von Acrylaten oder Methacrylaten von $C_{1-4}$-Alkoholen und Acrylaten oder Methacrylaten von $C_{8-30}$-Alkoholen,
     - (c) den gepfropften oder sequentiellen Blockcopolymeren, die mindestens einen sich bei der Polymerisation von Dienen ergebenden, hydrierten oder nicht hydrierten Block und mindestens einen Block eines Vinyl- oder Acrylpolymers oder eines Polyethers oder eines Polyesters aufweisen, oder deren Gemischen.